# EUROPEAN PATENT SPECIFICATION

(11) **EP 3 836 991 B1**
(45) Date of publication and mention of the grant of the patent: **13.11.2024**
(21) Application number: 19756139.2
(22) Date of filing: 13.08.2019
(51) Int. Cl.: A61M 5/31, A61M 5/32, B01D 63/08, G01N 30/18

(54) **FILTER APPARATUS FOR USE IN AN AUTOMATED SYSTEM FOR THE PREPARATION OF A SAMPLE FOR A CHEMICAL OR COMPOSITION ANALYSIS**
FILTERVORRICHTUNG ZUR VERWENDUNG IN EINEM AUTOMATISIERTEN SYSTEM ZUR VORBEREITUNG EINER PROBE FÜR EINE CHEMISCHE ODER KOMPOSITIONELLE ANALYSE
APPAREIL DE FILTRATION POUR L'UTILISATION DANS UN SYSTÈME AUTOMATISÉ POUR LA PRÉPARATION D'UN ÉCHANTILLON POUR L'ANALYSE CHIMIQUE OU DE COMPOSITION

(30) Priority: 13.08.2018 CH 9842018
(43) Date of publication of application: 23.06.2021
(73) Proprietor: Accroma Labtec AG, 4132 Muttenz (CH)
(72) Inventor: GAFNER, Stefan, 4444 Rümlingen (CH); FEDERER, Beat, 3005 Bern (CH)
(74) Representative: Latscha Schöllhorn Partner AG
(86) International application number: PCT/EP2019/071640
(87) International publication number: WO 2020/035468

(56) References cited:
- WO-A1-90/14798
- CN-A- 103 432 796
- CN-A- 106 390 749
- CN-U- 201 500 480
- CN-U- 201 987 980
- US-A- 5 567 309

## Description

### Technical Field

The present invention relates to a filter apparatus for use in an automated system for the preparation of a sample for a chemical or composition analysis and more particularly to a filter apparatus usable in connection with an autosampler or, more generally, with a robotic device designed to collect samples form a sample source, for instance comprised in a sample tray or carousel, and to feed them to an analytical instrument.

Such filter apparatus can be used for instance in analysis laboratories where relatively delicate, complex and costly machines are employed which are designed to perform sophisticate chemical or composition measurements, such as liquid chromatographers, titrators, water analyzers or similar.

In particular, high-performance liquid chromatography (HPLC; formerly referred to as high-pressure liquid chromatography), or ultra performance liquid chromatography (UPLC), used to separate, identify, and quantify each component in a sample mixture, require that a fluid sample be filtered, lest the analysis machinery be harmed by residual particulates in the fluid sample.

Sample materials processed can take many forms and can be inorganic, organic or biological. In particular, the sample material can be a hard and/or dry and/or brittle material, but also a softer material containing moisture, as in a biological tissue, or a viscous flowing mass in general. Therefore, the filter apparatus according to the present invention can be advantageously employed not only in the chemical and bio-chemical industry, but for instance also in food industry, e.g., if the food sample is analysed for heavy metals and similar. The automated system according to the present invention can be adapted to specific requirements of different areas of application, such as construction materials, metallurgy, textiles, pharmaceuticals or environmental analysis.

The present invention also relates to an automated system for the preparation of a sample for a chemical or composition analysis, comprising an injection head for the injection of an amount of sample in a receptacle and a filter apparatus.

The present invention further relates to a method of automated preparation of a sample for a chemical or composition analysis by way of an automated system, wherein the automated system comprises an injection head for the injection of an amount of sample in a receptacle via a needle.

### Background Art

According to conventional practices of sample preparation, a sample can be collected from a storing position, wherein the sample is typically contained in a vial retained in a vial rack recess.

The vial in which the sample is contained needs then to be carefully opened in order to enable taking out the sample and its consequent introduction into an extraction device.

Separation processes aimed at making a representative sample of a given material suitable for a subsequent chemical analysis require a preliminary fine size-reduction and homogenisation. Laboratory sample preparation protocols, in fact, require that the sample be manipulated, for instance by heating, screening, grinding, mixing etc., up to a target point of dissolution.

The extraction device can be operated to bring samples into a homogenous pulverized state and to prepare them perfectly for either additional sample preparation steps, and/or for subsequent analysis steps.

Subsequent analysis steps can be separating analytical methods like high-performance liquid chromatography (HPLC), or ultra performance liquid chromatography (UPLC), used to separate, identify, and quantify each component in a sample mixture.

As already mentioned, particulates or solids should be removed from a suspension of a fluid sample preparation, prior to submission to analysis machinery because of their adverse effect on the lifetime thereof. Particulates can affect HPLC hardware (e.g. flow lines, rotary injection valves, and inlet frits) and gas chromatography (GC) inlets, columns, and detectors.

Filtration is one common method for removing particulates or solids from the suspension created by the manipulated sample. Centrifugation and sedimentation are also known.

For the purpose of filtering, syringes and syringe filters are known. These however do not allow a real automation of the sample preparation and are not compatible with high-throughput autosamplers. CN 201 987 980 U, CN 201 500 480 U, WO 90/14798 A1, CN 103 432 796 A relate to syringe filter assemblies.

Preassembled filtration devices, in the form of disposable units, are already in use which comprise a plunger moving within a vial chamber. A filtration membrane is attached to a bottom end of the plunger; a pre-attached cap closes a top end of the plunger and a reservoir is created therebetween. An unfiltered suspension containing the sample is placed in the chamber. By fitting the plunger into the suspension-filled chamber, and by consequently pressing the plunger through the suspension, a clean filtrate is forced into the reservoir of the plunger. Excess air is vented through venting means integrated in the cap. Once the filtration has been carried out but dully pushing the plunger into the vial chamber, the cap can be removed or pierced with a needle and a sample drawn off for manual injection into an analyser machine.

However, such preassembled filtration devices have several drawbacks. It is difficult to ensure a fluid-tight sliding of the plunger into the chamber, with the ensuing risk of actually letting some undesired particle pass into the plunger reservoir. Moreover, the filtration membrane attached to the bottom end of the plunger is inherently small in surface: by construction, the filtration membrane cannot extend beyond the dimension of the plunger, which, in its turn, is limited by the dimension of the chamber within which it is made slidable. This structural limitation to the extension of the surface of the filtration membrane brings about problems with clogging of the pores of the membrane, as they do not have the capacity of effectively keeping on blocking physically the particulates from the suspension while letting the fluid through.

Therefore, there is a need for a filter apparatus for use in an automated system for the preparation of a sample for a chemical or composition analysis or such a system allowing a highly efficient automatized handling of a substantial number of samples and, at the same time, guaranteeing that the filtering action proceeds unaffected for the whole sample amount such that the analysis machines receive safely measurable analyte.

The filter apparatus, in order to ensure high throughput of prepared samples to be fed to the analysis machines, should be able to cleanly process a relatively high amount of sample volume passing therethrough, at a compatible flow rate.

### Disclosure of the Invention

According to the invention this need is settled by a filter apparatus as it is defined by the features of independent claim 1, by an automated system as it is defined by the features of independent claim 13 and by a method of automated preparation of a sample for a chemical analysis by way of an automated system as it is defined by the features of independent claim 14. Preferred embodiments are subject of the dependent claims.

In particular, in one aspect the invention deals with a filter apparatus for use in an automated system for the preparation of a fluid sample for a chemical or composition analysis.

The sample can originally have any suitable form such as the form of a tablet or other essentially solid body, a powder, a gel, a paste, a fluid or the like, which is suitable to be then processed by a processing unit of the automated system and reduced into a fluid form substantially equivalent to a suspension which needs to be further filtered to obtain a clear, particulate-free filtrate product. The sample material can particularly involve a drug. Thereby, the term "drug" relates to a therapeutically active agent, also commonly called active pharmaceutical ingredient (API), as well as to a combination of plural such therapeutically active substances. The term also encompasses diagnostic or imaging agents, like for example contrast agents (e.g. MRI contrast agents), tracers (e.g. PET tracers) and hormones, that need to be administered in liquid form to the patient.

The chemical or compositional analysis can be or involve any suitable analysis such as, particularly, high performance liquid chromatography (HPLC) or ultra high performance liquid chromatography (UHPLC or UPLC). The automated system can comprise an autosampler, for instance consisting of a robotic device that can bring the sample to a sampling processing unit or station and/or that can dose the sample to a receptacle on a tray or a carousel or a vial rack for delivery to a machine adapted to carry out the chemical or composition analysis.

The filter apparatus comprises an inlet portion with a conduit ending in an entry port for receiving the sample in an unfiltered state typically provided by or within the automated system. A septum is held at and closing the entry port of the conduit of the inlet portion.

The filter apparatus further comprises an outlet portion with a channel for dispensing the sample in a filtered state to a receptacle; as well as an intermediate portion having a filter chamber in fluid connection with the conduit of the inlet portion and with the channel of the outlet portion and a filter layer.

The filter chamber of the intermediate portion has a diameter larger than a diameter of the conduit of the inlet portion and than a diameter of the channel of the outlet portion.

The filter layer of the intermediate portion extends through the filter chamber of the intermediate portion such that the conduit of the inlet portion is separated from the channel of the outlet portion by the filter layer. Thanks to the filter layer in the filter chamber, the sample is filtered and dispensed to the receptacle in the form of clean filtrate.

The filter apparatus can be a membrane filter or syringe membrane filter. Besides the filter layer it can be made of a plastic material such as, e.g., polypropylene (PP), polyethylene (PE) or polytetrafluoroethylene (PTFE).

The receptacle can particularly be a vial or a similar suitable container. The term "receptacle" can relate to any container suitable for receiving or holding the sample material during storage thereof. The term "vial" as used herein can relate to vials in the literal sense, i.e. a comparably small vessel or bottle, often used to store pharmaceutical products or pharmaceuticals or medications in liquid, powdered or capsuled form. The vial can be made of a sterilisable material such as glass or plastic such as, e.g., polypropylene.

The inlet portion can define a distal end of the filter apparatus and the outlet portion a proximal end thereof, wherein by distal it is meant the direction that in use if furthest away from the receptacle or vial; whereas proximal indicates the direction which in use is closest to the receptacle or vial. The inlet and outlet portions can particularly be opposed to each other. The septum can be adapted to be pierced by a needle of an injection head of the automated system for the injection of the unfiltered sample fluid. The terms "conduit" and "channel" can relate to identical or similar structures or features of the filter apparatus. They are mainly used herein for reasons of comprehensibility, in particular, in order that the structure of the inlet portion can be differentiated from the structure of the outlet portion. More specifically, the conduit and the channel can be straight or curved ducts having a constant or varying diameter.

Preferably, the inlet portion and the outlet portion substantially extend along a longitudinal axis and, transversal to the longitudinal axis, the intermediate portion is wider than the inlet portion and than the outlet portion.

The intermediate portion, the inlet portion and the outlet portion may be, for instance, substantially cylindrically shaped.

Preferably, the intermediate portion is configured to remain in a fixed position relative to the outlet portion while the unfiltered sample is filtered through the filtering layer. Such a configuration overcomes any construction complication and the proneness to clogging typical of filter vials according to the prior art, wherein a plunger provided with a filtering membrane is slidably movable within the body of a vial chamber filled with unfiltered liquid.

In one possible embodiment, the filter apparatus preferably comprises a distal piece with a flange section and a proximal piece with a flange section, wherein the inlet portion is formed in the distal piece, whereas the outlet portion is formed in the proximal piece. In such case, the distal piece and the proximal piece are preferably mounted to each other at the flange sections, and the flange section of the distal piece and the flange section of the proximal piece are configured to form the intermediate portion when the distal piece is mounted to the proximal piece. Hence, a simple and effective construction of the intermediate portion incorporating a filter chamber is given, also offering the possibility of easily affixing the filter layer thereto.

Preferably, in the configuration of the embodiment above described, the filter layer is clamped in between the distal piece and the proximal piece such that it extends across the filter chamber of the intermediate portion.

Moreover, the flange section of the distal piece may be provided with a distribution structure and the flange section of the proximal piece may be additionally provided with a distribution structure, such that the distal and proximal walls of the filter chamber are equipped with the distribution structures when the distal piece is mounted to the proximal piece. The distribution structures allow for an optimal coverage and penetration of the filter layer by the fluid sample, thanks to an enhanced, uniform spreading of the fluid sample over the whole surface of the filter layer available. No limited region of the filter layer is thus overly burdened by the passing fluid sample.

Preferably, the filter chamber is substantially liquid-tight. This feature also guarantees that no portion of the fluid sample circumvents the filter layer and is left unfiltered.

A recess of the flange section of the proximal piece and/or a recess of the flange section of the distal piece may comprise a further filtering element adapted to cooperate with the filter layer. A further filtering element could for instance be conceived to preliminarily capture bigger particles upstream of the filter layer and/or refine the filtrate in a targeted way. Additional filtering elements can also be incorporated or embedded in the above described distribution structures, for instance in case the suspension of the fluid sample is particularly dense.

Preferably, the filter layer has a surface area adapted to allow a complete filtration passage of the unfiltered fluid sample received in the conduit of the inlet portion in a target time. In order to improve a throughput of the automated system, the target time within which all of the fluid sample has passed through the filter layer is advantageously minimized. The relatively large area of the filter layer configured as previously described enables a quick passage of the fluid sample.

Preferably, the surface area of the filter layer is in a range of about 300 mm² to about 400 mm² and/or the filter layer has a porosity in a range of about 0.02 µm to about 40 µm.

Preferably, the outlet portion has a dispensing nozzle, the channel ends the dispensing nozzle and the dispensing nozzle is configured to prevent the formation of drops. Thus, a clean and lossless delivery of the filtered fluid sample to an underlying receptacle, such as a vial, is guaranteed.

Preferably, the filter layer comprises a filter membrane. The filter membrane can be made of polyethersulfone (PES), regenerated cellulose (RC) or polyvinylidene fluoride.

Preferably, the filter chamber of the intermediate portion extends transversal to a direction of flow defined by the conduit of the inlet portion and the channel of the outlet portion.

In order to enable a fast, complete and unobstructed passage of the fluid sample through the filter layer therein contained, the diameter of the filter chamber of the intermediate portion is at least two times, at least three times, at least four times or at least five times larger than the diameter of the conduit of the inlet portion and than the diameter of the channel of the outlet portion.

The present invention also relates to an automated system for the preparation of a sample for a chemical or composition analysis, comprising an injection head -also designatable as dosing apparatus- for the injection -or dosage- of an amount of sample in a receptacle and a filter apparatus according to any one of the preceding claims. The injection head is equipped with a needle adapted to be affixed to the filter apparatus by piercing a septum of the filter apparatus.

The filter apparatus can be disposable and affixable to the needle of the injection head, or dosing apparatus, every time that a fluid sample needs to be newly filtered. Alternatively, the same filter apparatus affixed to one needle of the injection head may be employed for successively distributing a sample fluid into a series of different receptacles.

The present invention also relates to a method of automated preparation of a sample for a chemical analysis by way of an automated system, wherein the automated system comprises an injection head for the injection of an amount of sample in a receptacle via a needle.

The method of automated preparation of a sample for a chemical analysis according to the present invention comprises the steps of obtaining a filter apparatus as above described and of preventively affixing the needle by piercing a septum of the filter apparatus. The method further comprises the steps of positioning the filter apparatus adjacent to a receptacle and establishing a fluid connection between the needle of the injection head and the receptacle through the filter apparatus.

The filter apparatus is preferably designed as disposable, for instance being designed for just a single use. There can also be envisaged the possibility to design the filter apparatus such that the need for substitution thereof first arises after a programmed number of pipetting injection cycles, whether executed in one same receptacle or in a series of distinct receptacles.

### Brief Description of the Drawings

The filter apparatus and the automated system for the preparation of a sample for a chemical or composition analysis adapted to employ such filter apparatus according to the invention are described in more detail herein below by way of an exemplary embodiment and with reference to the attached drawings, in which:
Fig. 1 shows a side view of a possible embodiment of a filter apparatus according to the present invention;
Fig. 2 shows a section view A-A of the filter apparatus of Fig. 1; and
Figs. 3a-3c show, respectively, a top view, a bottom view and a lateral view of a distal piece with a flange section which is part of the filter apparatus of Fig. 1;
Figs. 4a-4c show, respectively, a top view, a bottom view and a lateral view of a proximal piece with a flange section configured to be mounted to the distal piece of Figs. 3a-3c;
Fig. 5 shows a perspective view of an automated system for the preparation of a sample for a chemical or composition analysis according to the present invention, adapted to employ the filter apparatus of Fig. 1; and
Fig. 6 shows a view from top of the automated system of Fig. 5.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

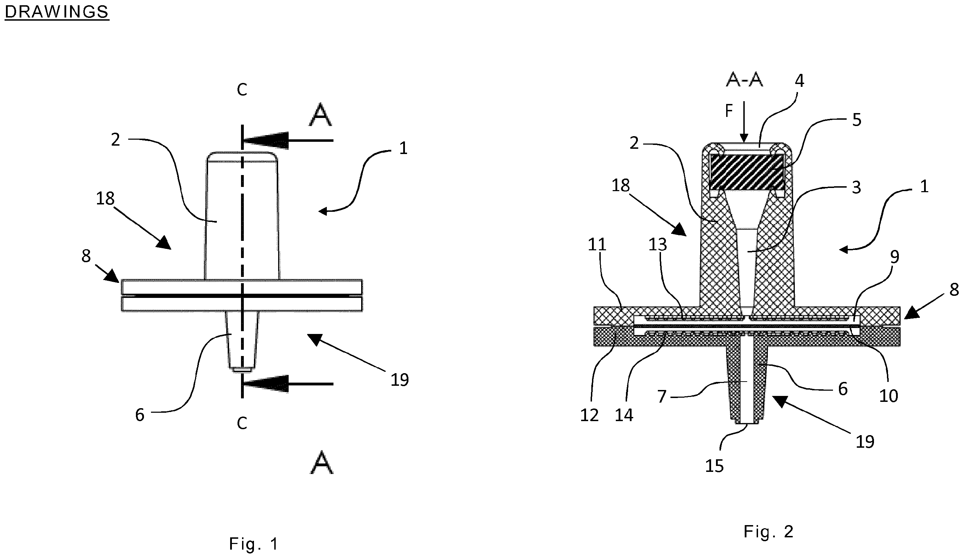

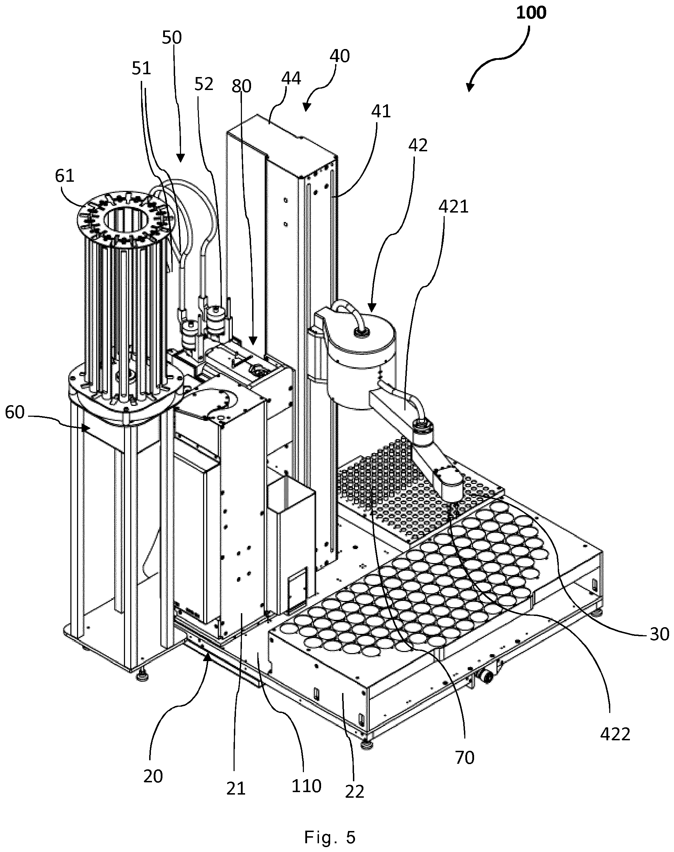

With reference to Figs. 1 and 5, a filter apparatus 1 for use in an automated system 100 for the preparation of a sample for a chemical or composition analysis according to the present invention comprises an inlet portion 2, an outlet portion 6 and an intermediate portion 8.

As shown represented in Fig. 2, the inlet portion 2 comprises a conduit 3 ending in an entry port 4 for receiving an unfiltered fluid sample delivered by the automated system 100. In this specific case, the conduit 3 comprises a funnel-shaped section to better collect and direct the dosed fluid.

The outlet portion 6 comprises a channel 7 for dispensing the filtered sample to a receptacle 70, such as a vial. In the represented instance, the channel 7 has a substantially constant cylindrical section.

A septum 5 is held at, and closes, the entry port 4 of the conduit 3 of the inlet portion 2.

Also in the section view of Fig. 2, it is exemplified how the substantially cylindrical intermediate portion 8 has a filter chamber 9 which is in fluid connection with the conduit 3 of the inlet portion 2 and with the channel 7 of the outlet portion 6.

The filter chamber 9 of the intermediate portion 8 extends transversal to a direction of flow F defined by the conduit 3 of the inlet portion 2 and the channel 7 of the outlet portion 6.

The inlet portion 2 and the outlet portion 6 substantially extend along a longitudinal axis C-C. Transversal to the longitudinal axis C-C, the intermediate portion 8 is wider than the inlet portion 2 and than the outlet portion 6.

A filter layer 10, in the form of a membrane, extends through the filter chamber 9 such that the conduit 3 of the inlet portion 2 is separated from the channel 7 of the outlet portion 6 by the filter layer 10.

Thus, the fluid sample, having been dosed into the entry port 4, is conveyed via the conduit 3 to the liquid-tight filter chamber 9 and, once completely filtered by the filter layer 10, is then fully carried through the channel 7 to be ultimately dispensed to a receptacle 70.

For the dispensing purpose, the outlet portion 6 is provided with a dispensing nozzle 15, the channel 7 ending the dispensing nozzle 15. The dispensing nozzle 15 is aptly configured to prevent the formation of drops.

The diameter of the filter chamber 9 of the intermediate portion 8 is larger than the diameter of the widest section of the funnel-shaped conduit 3 of the inlet portion 2 and than the diameter of the channel 7 of the outlet portion 6.

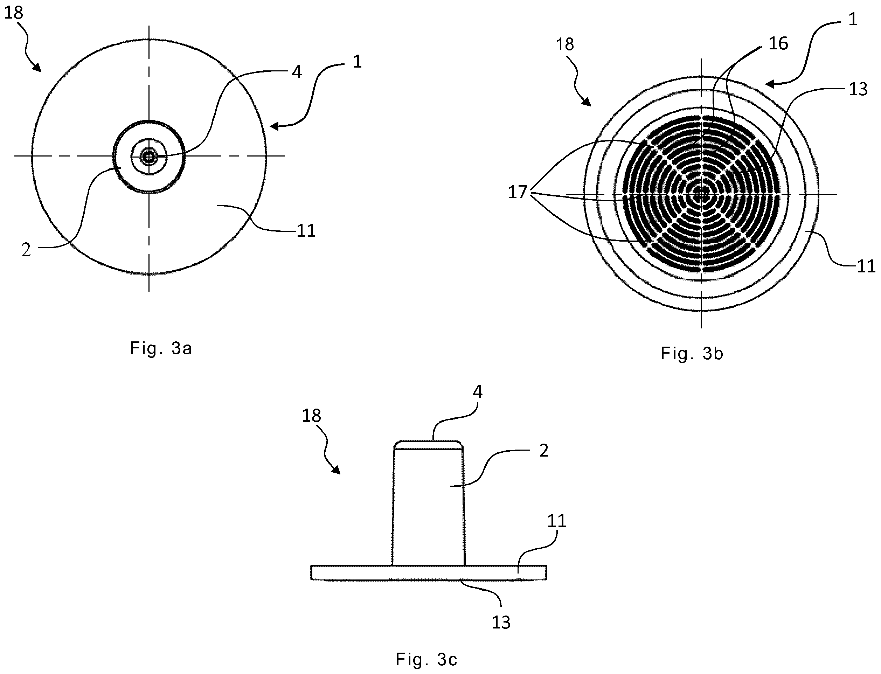

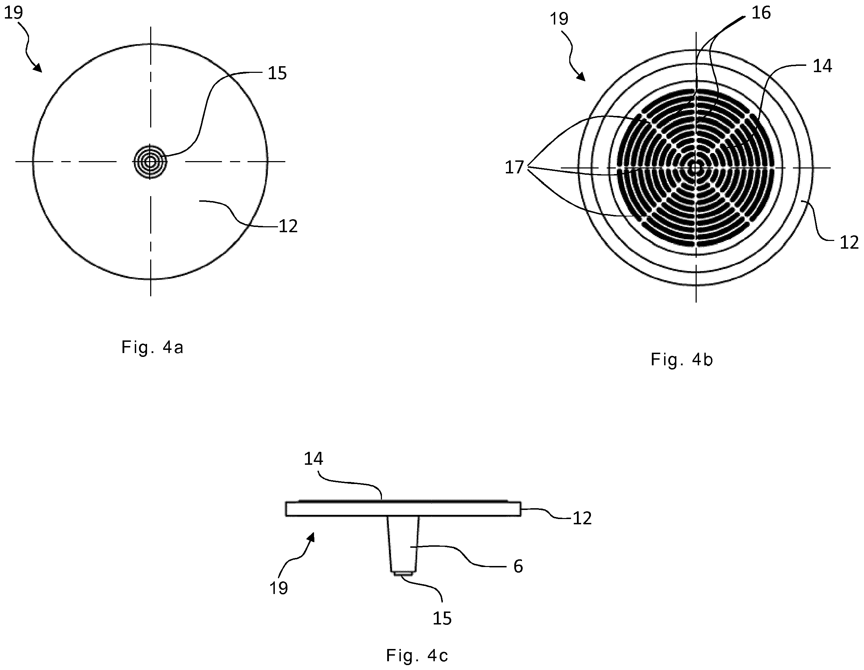

As it can be appreciated from Figs. 2, 3c and 4c, in the present embodiment the inlet portion 2 is formed in a distal piece 18, whereas the outlet portion 6 is formed in a proximal piece 19. The distal piece 18 is formed with a flange section 11 and the proximal piece 19 with a flange section 12. The distal piece 18 and the proximal piece 19 are mounted to each other at the flange sections 11, 12, such that the intermediate portion 8 is formed in the mounted configuration. In the embodiment of Fig. 2, the filter chamber 9 is created by two matching recesses respectively in the flange section 11 and in the flange section 12.

In such mounted configuration, the filter layer 10 is clamped in between the distal piece 18 and the proximal piece 19 such that it extends across the filter chamber 9 of the intermediate portion 8.

As represented in Figs, 2, 3b and 4b, the flange section 11 of the distal piece 18 has a distribution structure 13 and the flange section 12 of the proximal piece 19 has a distribution structure 14. Thus, the distal and proximal walls of the filter chamber 9 are equipped with the distribution structures 13, 14, when the distal piece 18 is mounted to the proximal piece 19. In the specific embodiment portrayed, the distribution structures 13, 14 take the form of wedge-shaped circular sectors integrating concentric distribution grooves 16. Each circular sector is separated from adjacent circular sectors by radial grooves 17 opening into each of the concentric distribution grooves 16. Thus, grooves 16 and 17 form a network of distribution sulci which guarantee a uniform spreading of the fluid sample to be filtered over the whole surface of the filter layer 10.

The intermediate portion 8 is configured to remain in a fixed position relative to the outlet portion 6 while the unfiltered sample is filtered through the filter layer 10.

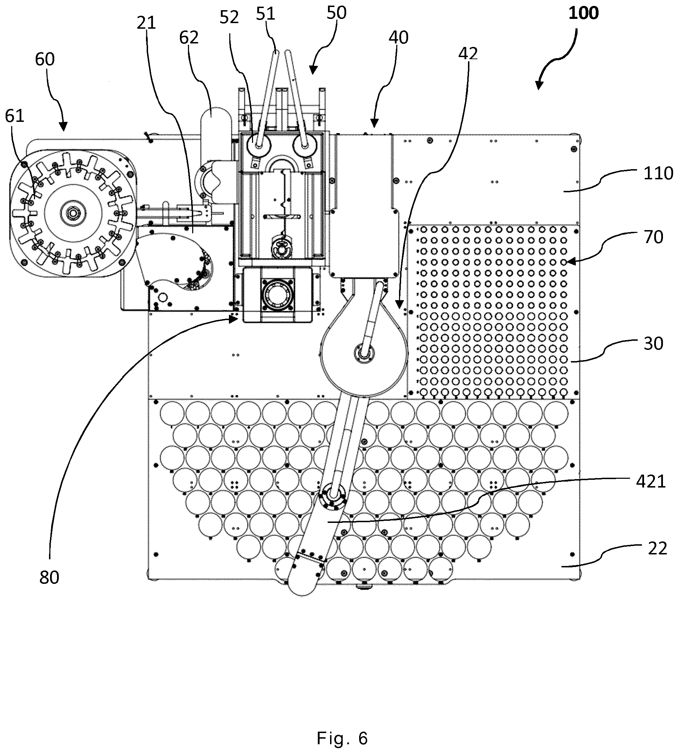

The present invention also relates to an automated system 100 for the preparation of a sample for a chemical or composition analysis, comprising an injection head 50 for the injection of an amount of sample in a receptacle 70 and a filter apparatus 1 as above described. A possible embodiment for such an automated system is represented in Figs. 5 and 6.

The automated system 100 comprises a handling robot 40; a ball mill apparatus 20; an injection head 50, also designatable as dosing apparatus 50; a filter dispenser 60 and a sample processing unit 80.

The automated system 100 is equipped with a platform 110 on which several components are positioned. The platform 110 has height adjustable feet which allow for aligning the platform 110 such that it lies in an essentially horizontal plane.

The handling robot 40 has a vertical stand 44 which is equipped with vertically extending parallel rails 41. On the rails 41 an arm unit 42 is slidably mounted such that the arm unit 42 can vertically move along the rails 41. The arm unit 42 has a cylinder portion, which is pivotable about a vertical axis and an arm 421 with joints ending in gripping fingers 422.

The ball mill apparatus 20 has an agitating station 21 which is positioned at a left hand side of the platform 110. It further has an extraction tube rack 22 which extends along the complete front side of the platform 110. The dosing apparatus 50 and the sample processing unit 80 are positioned between the agitating station 21 of the ball mill apparatus 20 and the stand 44 of the handling robot 40. The dosing apparatus 50 has two needles 51 and two sucking pumps 52, wherein each pump 52 is coupled to one of the needles 51 by a flexible tube.

In Fig. 6 the automated system 100 is shown top down. Thereby, it can be seen that the filter dispenser 60 has a filter storage 61 positioned left hand aside the platform 110 and a filter disposer 62 positioned left hand aside the dosing apparatus 50. The extraction tube rack 22 forms the front of the automated system 100 and the other components, i.e. the agitating station 21, the dosing apparatus 50, the sample processing unit 80, the handling robot 40 are arranged behind the extraction tube rack 22.

A vial rack 30 is also arranged behind the extraction tube rack 22, on platform 110. The vial rack 30 has recesses in which glass vials 70 are received as receptacles. The vials 70 are meant to house the fluid sample once filtered, that is ready for analysis, e.g. by HPLC.

The handling robot 40 takes a container of fluid sample, possibly already partially processed by the sample processing unit 80, with its gripping fingers 422 and places it into one of the extraction tubes pre-positioned in the extraction tube rack 22. The handling robot 40 then relocates the extraction tube into the agitating station 21 of the ball mill apparatus 20. There the sample material is grinded thanks to the movement imparted by a drive agitating the extraction tube and in particular by grinding balls.

After the sample has been grinded, the extraction tube is relocated into the extraction tube rack 22 where it is ready for further preparation by the sample processing unit 80.

The fluid sample, thus broken-down and reduced by the agitating station 21 and processed by the sample processing unit 80, is ready to be sucked by the injection head, or dosing apparatus, 50 via needles 51 under the sucking action of pumps 52.

At least one of the needles 51 is adapted to be affixed to a filter apparatus 1 which is summoned from the filter storage 61 of the filter dispenser 60 and pre-arranged by the filter disposer 62 for the needle 51 to pierce its septum 5.

The affixing of the needle 51 to the filter apparatus 1 is obtained by the needle 51 piercing a septum 5 of the filter apparatus 1.

The injection head 50 positions then the filter apparatus 1 adjacent to a receptacle 70, such as a vial, and a fluid connection is established between the needle 51 of the injection head 50 and the vial 70 through the filter apparatus 1.

The prepared fluid sample material is thus transferred from the injection head 50 to the vial 70 through the filter apparatus 1 provided by the filter dispenser 60, so that it can be then analysed in an appropriate HPLC equipment.

As mentioned, the filter apparatus 1 is preferably disposable. However, the same filter apparatus 1 can be employed for establishing a fluid connection between the needle 51 of the injection head 50 and a series of different receptacles 70, distributing one filtrate into multiple receptacles. The filter apparatus 1 can be designed to be substituted only after a programmed number of injection cycles, whether the injection or dosing happens in just one receptacle 70 or is distributed into several distinct receptacles 70.

This description and the accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfill the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. A filter apparatus (1) for use in an automated system (100) for the preparation of a fluid sample for a chemical or composition analysis, comprising:
an inlet portion (2) with a conduit (3) ending in an entry port (4) for receiving the sample in an unfiltered state;
a septum (5) held at and closing the entry port (4) of the conduit (3) of the inlet portion (2);
an outlet portion (6) with a channel (7) for dispensing the sample in a filtered state to a receptacle (70); and
an intermediate portion (8) having a filter chamber (9) in fluid connection with the conduit (3) of the inlet portion (2) and with the channel (7) of the outlet portion (6), and a filter layer (10); wherein
the filter chamber (9) of the intermediate portion (8) has a diameter larger than a diameter of the conduit (3) of the inlet portion (2) and than a diameter of the channel (7) of the outlet portion (6), and
the filter layer (10) extends through the filter chamber (9) such that the conduit (3) of the inlet portion (2) is separated from the channel (7) of the outlet portion (6) by the filter layer (10).

2. The filter apparatus (1) of claim 1, wherein the inlet portion (2) and the outlet portion (6) substantially extend along a longitudinal axis (C-C) and, transversal to the longitudinal axis (C-C), the intermediate portion (8) is wider than the inlet portion (2) and than the outlet portion (6) and wherein preferably the intermediate portion (8), the inlet portion (2) and the outlet portion (6) are substantially cylindrically shaped.

3. The filter apparatus (1) of any one of the preceding claims, wherein the intermediate portion (8) is configured to remain in a fixed position relative to the outlet portion (6) while the sample is filtered through the filter layer (10).

4. The filter apparatus (1) of any one of the preceding claims, comprising a distal piece (18) with a flange section (11) and a proximal piece (19) with a flange section (12), wherein
the inlet portion (2) is formed in the distal piece (18), the outlet portion (6) is formed in the proximal piece (19),
the distal piece (18) and the proximal piece (19) are mounted to each other at the flange sections (11, 12),
the flange section (11) of the distal piece (18) and the flange section (12) of the proximal piece (19) form the intermediate portion (8) and
the filter layer (10) preferably is clamped in between the distal piece (18) and the proximal piece (19) such that it extends across the filter chamber (9) of the intermediate portion (8).

5. The filter apparatus (1) of claim 4, wherein the flange section (11) of the distal piece (18) has a distribution structure (13) and the flange section (12) of the proximal piece (19) has a distribution structure (14) such that the distal and proximal walls of the filter chamber (9) are equipped with the distribution structures (13, 14).

6. The filter apparatus (1) of any one of the preceding claims, wherein the filter chamber (9) is liquid-tight.

7. The filter apparatus (1) of any one of the preceding claims, wherein the filter layer (10) has a surface area adapted to allow a complete filtration passage of the sample received in the conduit (3) of the inlet portion (2) in a target time and wherein preferably the surface area of the filter layer (10) is in a range of about 300 mm² to about 400 mm² and/or the filter layer has a porosity in a range of about 0.02 µm to about 40 µm.

8. The filter apparatus (1) of any one of the preceding claims, wherein the outlet portion (6) has a dispensing nozzle (15) and the channel (7) ends the dispensing nozzle (15).

9. The filter apparatus (1) of any one of the preceding claims, wherein the filter layer (10) comprises a filter membrane.

10. The filter apparatus (1) of any one of the preceding claims, wherein the filter chamber (9) of the intermediate portion (8) extends transversal to a direction of flow (F) defined by the conduit (3) of the inlet portion (2) and the channel (7) of the outlet portion (6).

11. The filter apparatus (1) of any one of claims 2 to 10, wherein the direction of flow (F) extends essentially along the longitudinal axis (C-C).

12. The filter apparatus (1) of any one of the preceding claims, wherein the diameter of the filter chamber (9) of the intermediate portion (8) is at least two times, at least three times, at least four times or at least five times larger than the diameter of the conduit (3) of the inlet portion (2) and than the diameter of the channel (7) of the outlet portion (6).

13. An automated system (100) for the preparation of a fluid sample for a chemical or composition analysis, comprising an injection head (50) for the injection of an amount of sample in a receptacle (70) and a filter apparatus (1) according to any one of the preceding claims, wherein the injection head (50) is equipped with a needle (51) adapted to be affixed to the filter apparatus (1) by piercing a septum (5) of the filter apparatus (1 and wherein preferably the same filter apparatus (1) affixed to one needle (51) of the injection head (50) is employed for pipetting of one sample fluid into a series of distinct receptacles (70).

14. A method of automated preparation of a fluid sample for a chemical analysis by way of an automated system (100), wherein the automated system (100) comprises an injection head (50) for the injection of an amount of the sample in a receptacle (70) via a needle (51),
comprising the steps of
obtaining a filter apparatus (1) according to any one of claims 1 to 13,
preventively affixing the needle (51) by piercing a septum (5) of the filter apparatus (1);
positioning the filter apparatus (1) adjacent to a receptacle (70); establishing a fluid connection between the needle (51) of the injection head (50) and the receptacle (70) through the filter apparatus (1).

15. The method of claim 14, 4-8,- comprising the step of substituting the filter apparatus (1) after a programmed number of injection cycles.

## Patentansprüche

1. Filtervorrichtung (1) zur Verwendung in einem automatisierten System (100) zum Zubereiten einer Flüssigkeitsprobe für eine chemische Analyse oder eine Analyse der Zusammensetzung, umfassend:
einen Einlassabschnitt (2) mit einer Leitung (3), die in einer Eingangsöffnung (4) endet, um die Probe in einem ungefiltertem Zustand aufzunehmen;
ein Septum (5), das an der Eingangsöffnung (4) der Leitung (3) des Einlassabschnitts (2) gehalten wird und diese verschließt;
einen Auslassabschnitt (6) mit einem Kanal (7) zum Abgeben der Probe in gefiltertem Zustand in einen Behälter (70); und
einen Zwischenabschnitt (8) mit einer Filterkammer (9), die in Flüssigkeitsverbindung mit der Leitung (3) des Einlassabschnitts (2) und mit dem Kanal (7) des Auslassabschnitts (6) steht, sowie einer Filterschicht (10); wobei
die Filterkammer (9) des Zwischenabschnitts (8) einen Durchmesser aufweist, der größer ist als ein Durchmesser der Leitung (3) des Einlassabschnitts (2) und als ein Durchmesser des Kanals (7) des Auslassabschnitts (6), und
die Filterschicht (10) sich durch die Filterkammer (9) erstreckt, so dass die Leitung (3) des Einlassabschnitts (2) durch die Filterschicht (10) von dem Kanal (7) des Auslassabschnitts (6) getrennt ist.

2. Filtervorrichtung (1) nach Anspruch 1, wobei sich der Einlassabschnitt (2) und der Auslassabschnitt (6) im Wesentlichen entlang einer Längsachse (C-C) erstrecken und der Zwischenabschnitt (8) quer zur Längsachse (C-C) breiter als der Einlassabschnitt (2) und als der Auslassabschnitt (6) ist, und wobei vorzugsweise der Zwischenabschnitt (8), der Einlassabschnitt (2) und der Auslassabschnitt (6) im Wesentlichen zylindrisch geformt sind.

3. Filtervorrichtung (1) nach einem der vorstehenden Ansprüche, wobei der Zwischenabschnitt (8) so konfiguriert ist, dass er in einer festen Position relativ zum Auslassabschnitt (6) verbleibt, während die Probe durch die Filterschicht (10) gefiltert wird.

4. Filtervorrichtung (1) nach einem der vorstehenden Ansprüche, umfassend ein distales Teil (18) mit einem Flanschabschnitt (11) und ein proximales Teil (19) mit einem Flanschabschnitt (12), wobei
der Einlassabschnitt (2) im distalen Teil (18) ausgebildet ist, der Auslassabschnitt (6) im proximalen Teil (19) ausgebildet ist,
das distale Teil (18) und das proximale Teil (19) an den Flanschabschnitten (11, 12) miteinander verbunden sind,
der Flanschabschnitt (11) des distalen Teils (18) und der Flanschabschnitt (12) des proximalen Teils (19) den Zwischenabschnitt (8) bilden und
die Filterschicht (10) vorzugsweise zwischen dem distalen Teil (18) und dem proximalen Teil (19) so eingeklemmt ist, dass sie sich über die Filterkammer (9) des Zwischenabschnitts (8) erstreckt.

5. Filtervorrichtung (1) nach Anspruch 4, wobei der Flanschabschnitt (11) des distalen Teils (18) eine Verteilungsstruktur (13) aufweist und der Flanschabschnitt (12) des proximalen Teils (19) eine Verteilungsstruktur (14) aufweist, so dass die distalen und proximalen Wände der Filterkammer (9) mit den Verteilungsstrukturen (13, 14) ausgestattet sind.

6. Filtervorrichtung (1) nach einem der vorstehenden Ansprüche, wobei die Filterkammer (9) flüssigkeitsdicht ist.

7. Filtervorrichtung (1) nach einem der vorstehenden Ansprüche, wobei die Filterschicht (10) eine Oberfläche aufweist, die so angepasst ist, dass sie einen vollständigen Filtrationsdurchgang der in der Leitung (3) des Einlassabschnitts (2) aufgenommenen Probe in einer Zielzeit ermöglicht, und wobei die Oberfläche der Filterschicht (10) vorzugsweise in einem Bereich von etwa 300 mm² bis etwa 400 mm² liegt und/oder die Filterschicht eine Porosität im Bereich von etwa 0,02 µm bis etwa 40 µm aufweist.

8. Filtervorrichtung (1) nach einem der vorstehenden Ansprüche, wobei der Auslassabschnitt (6) eine Abgabedüse (15) aufweist und der Kanal (7) an der Abgabedüse (15) endet.

9. Filtervorrichtung (1) nach einem der vorstehenden Ansprüche, wobei die Filterschicht (10) eine Filtermembran umfasst.

10. Filtervorrichtung (1) nach einem der vorstehenden Ansprüche, wobei sich die Filterkammer (9) des Zwischenabschnitts (8) quer zu einer durch die Leitung (3) des Einlassabschnitts (2) und den Kanal (7) des Auslassabschnitts (6) definierten Strömungsrichtung (F) erstreckt.

11. Filtervorrichtung (1) nach einem der Ansprüche 2 bis 10, wobei die Strömungsrichtung (F) im Wesentlichen entlang der Längsachse (C-C) verläuft.

12. Filtervorrichtung (1) nach einem der vorstehenden Ansprüche, wobei der Durchmesser der Filterkammer (9) des Zwischenabschnitts (8) mindestens zwei Mal, mindestens drei Mal, mindestens vier Mal oder mindestens fünf Mal größer ist als der Durchmesser der Leitung (3) des Einlassabschnitts (2) und als der Durchmesser des Kanals (7) des Auslassabschnitts (6).

13. Automatisiertes System (100) zum Zubereiten einer Flüssigkeitsprobe für eine chemische Analyse oder einer Analyse der Zusammensetzung, umfassend einen Injektionskopf (50) zum Injizieren einer Probenmenge in einen Behälter (70) und eine Filtervorrichtung (1) nach einem der vorstehenden Ansprüche, wobei der Injektionskopf (50) mit einer Nadel (51) ausgestattet ist, die an der Filtervorrichtung (1) durch Durchstechen eines Septums (5) der Filtervorrichtung (1) befestigt werden kann, und wobei vorzugsweise dieselbe Filtervorrichtung (1), die an einer Nadel (51) des Injektionskopfs (50) befestigt ist, zum Pipettieren einer Probenflüssigkeit in eine Reihe unterschiedlicher Behälter (70) verwendet wird.

14. Verfahren zum automatisierten Zubereiten einer Flüssigkeitsprobe für eine chemische Analyse mittels eines automatisierten Systems (100), wobei das automatisierte System (100) einen Injektionskopf (50) zum Injizieren einer Menge der Probe in einen Behälter (70) mittels einer Nadel (51) umfasst,
umfassend die Schritte
Erhalten einer Filtervorrichtung (1) nach einem der Ansprüche 1 bis 13,
vorbeugendes Fixieren der Nadel (51) durch Durchstechen eines Septums (5) der Filtervorrichtung (1);
Positionieren der Filtervorrichtung (1) neben einem Behälter (70);
Herstellen einer Flüssigkeitsverbindung zwischen der Nadel (51) des Injektionskopfes (50) und dem Behälter (70) durch die Filtervorrichtung (1).

15. Verfahren nach Anspruch 14, umfassend den Schritt des Auswechselns der Filtervorrichtung (1) nach einer programmierten Anzahl von Injektionszyklen.

## Revendications

1. Appareil de filtration (1) destiné à être utilisé dans un système automatisé (100) pour la préparation d'un échantillon de fluide en vue d'une analyse chimique ou de composition, comprenant :
une partie d'entrée (2) avec un conduit (3) se terminant dans un orifice d'entrée (4) pour recevoir l'échantillon à l'état non filtré ;
un septum (5) maintenu au niveau du et fermant l'orifice d'entrée (4) du conduit (3) de la partie d'entrée (2) ;
une partie de sortie (6) avec un canal (7) pour distribuer l'échantillon à l'état filtré dans un réceptacle (70) ; et
une partie intermédiaire (8) ayant une chambre de filtration (9) en liaison fluidique avec le conduit (3) de la partie d'entrée (2) et avec le canal (7) de la partie de sortie (6), et une couche de filtration (10) ; dans lequel
la chambre de filtration (9) de la partie intermédiaire (8) a un diamètre supérieur à un diamètre du conduit (3) de la partie d'entrée (2) et à un diamètre du canal (7) de la partie de sortie (6), et
la couche de filtration (10) s'étend à travers la chambre de filtration (9) de telle sorte que le conduit (3) de la partie d'entrée (2) est distinct du canal (7) de la partie de sortie (6) par la couche de filtration (10).

2. Appareil de filtration (1) selon la revendication 1, dans lequel la partie d'entrée (2) et la partie de sortie (6) s'étendent sensiblement le long d'un axe longitudinal (C-C) et, transversalement à l'axe longitudinal (C-C), la partie intermédiaire (8) est plus large que la partie d'entrée (2) et que la partie de sortie (6) et dans lequel, de préférence, la partie intermédiaire (8), la partie d'entrée (2) et la partie de sortie (6) sont sensiblement de forme cylindrique.

3. Appareil de filtration (1) selon l'une quelconque des revendications précédentes, dans lequel la partie intermédiaire (8) est conçue pour rester dans une position fixe par rapport à la partie de sortie (6) pendant que l'échantillon est filtré à travers la couche de filtration (10).

4. Appareil de filtration (1) selon l'une quelconque des revendications précédentes, comprenant une pièce distale (18) avec une section de bride (11) et une pièce proximale (19) avec une section de bride (12), dans lequel
la partie d'entrée (2) est formée dans la pièce distale (18), la partie de sortie (6) est formée dans la pièce proximale (19),
la pièce distale (18) et la pièce proximale (19) sont montées l'une sur l'autre au niveau des sections de bride (11, 12),
la section de bride (11) de la pièce distale (18) et la section de bride (12) de la pièce proximale (19) forment la partie intermédiaire (8) et
la couche de filtration (10) est de préférence serrée entre la pièce distale (18) et la pièce proximale (19) de telle sorte qu'elle s'étend sur l'ensemble de la chambre de filtration (9) de la partie intermédiaire (8).

5. Appareil de filtration (1) selon la revendication 4, dans lequel la section de bride (11) de la pièce distale (18) a une structure de distribution (13) et la section de bride (12) de la pièce proximale (19) a une structure de distribution (14) de telle sorte que les parois distales et proximales de la chambre de filtration (9) sont équipées des structures de distribution (13, 14).

6. Appareil de filtration (1) selon l'une quelconque des revendications précédentes, dans lequel la chambre de filtration (9) est étanche aux liquides.

7. Appareil de filtration (1) selon l'une quelconque des revendications précédentes, dans lequel la couche de filtration (10) a une surface adaptée à permettre un passage de filtration complet de l'échantillon reçu dans le conduit (3) de la partie d'entrée (2) pendant une durée cible et dans lequel, de préférence, la surface de la couche de filtration (10) est comprise entre environ 300 mm² et environ 400 mm² et/ou la couche de filtration a une porosité comprise entre environ 0,02 µm et environ 40 µm.

8. Appareil de filtration (1) selon l'une quelconque des revendications précédentes, dans lequel la partie de sortie (6) a une buse de distribution (15) et le canal (7) se termine par la buse de distribution (15).

9. Appareil de filtration (1) selon l'une des revendications précédentes, dans lequel la couche de filtration (10) comprend une membrane de filtration.

10. Appareil de filtration (1) selon l'une quelconque des revendications précédentes, dans lequel la chambre de filtration (9) de la partie intermédiaire (8) s'étend transversalement à une direction d'écoulement (F) définie par le conduit (3) de la partie d'entrée (2) et le canal (7) de la partie de sortie (6).

11. Appareil de filtration (1) selon l'une quelconque des revendications 2 à 10, dans lequel la direction de l'écoulement (F) s'étend essentiellement le long de l'axe longitudinal (C-C).

12. Appareil de filtration (1) selon l'une quelconque des revendications précédentes, dans lequel le diamètre de la chambre de filtration (9) de la partie intermédiaire (8) est au moins deux fois, au moins trois fois, au moins quatre fois ou au moins cinq fois plus grand que le diamètre du conduit (3) de la partie d'entrée (2) et que le diamètre du canal (7) de la partie de sortie (6).

13. Système automatisé (100) destiné à la préparation d'un échantillon de fluide pour une analyse chimique ou de composition, comprenant une tête d'injection (50) pour l'injection d'une quantité d'échantillon dans un réceptacle (70) et appareil de filtration (1) selon l'une quelconque des revendications précédentes, dans lequel la tête d'injection (50) est équipée d'une aiguille (51) adaptée à être fixée à l'appareil de filtration (1) en perçant un septum (5) de l'appareil de filtration (1) et dans lequel, de préférence, le même appareil de filtration (1) fixé à une aiguille (51) de la tête d'injection (50) est utilisé pour le pipetage d'un échantillon de fluide dans une série de réceptacles distincts (70).

14. Procédé de préparation automatisée d'un échantillon de fluide pour une analyse chimique au moyen d'un système automatisé (100), dans lequel le système automatisé (100) comprend une tête d'injection (50) pour l'injection d'une quantité de l'échantillon dans un réceptacle (70) par l'intermédiaire d'une aiguille (51),
comprenant les étapes consistant à :
obtenir un appareil de filtration (1) selon l'une quelconque des revendications 1 à 13,
fixer de manière préventive l'aiguille (51) en perçant un septum (5) de l'appareil de filtration (1) ;
positionner l'appareil de filtration (1) adjacent à un réceptacle (70) ;
établir une liaison fluidique entre l'aiguille (51) de la tête d'injection (50) et le réceptacle (70) à travers l'appareil de filtration (1).

15. Procédé selon la revendication 14, comprenant l'étape consistant à remplacer l'appareil de filtration (1) après un nombre programmé de cycles d'injection.
